# EUROPÄISCHE PATENTSCHRIFT

(11) **EP 2 041 081 B1**
(45) Veröffentlichungstag und Bekanntmachung des Hinweises auf die Patenterteilung: **15.09.2010**
(21) Anmeldenummer: 07730218.0
(22) Anmeldetag: 18.06.2007
(51) Int. Cl.: C07C 407/00, C07C 409/32, C07C 409/38, B01J 19/18

(54) **KONTINUIERLICHES VERFAHREN ZUR HERSTELLUNG VON ACYLPEROXIDEN**
CONTINUOUS METHOD FOR PRODUCING ACYL PEROXIDES
PROCÉDÉ CONTINU DE PRODUCTION DE PEROXYDES D'ACYLE

(30) Priorität: 12.07.2006 DE 102006032165
(43) Veröffentlichungstag der Anmeldung: 01.04.2009
(73) Patentinhaber: United Initiators GmbH & Co. KG, 82049 Pullach (DE)
(72) Erfinder: APPEL, Hans, 82377 Penzberg (DE); MEICHELBÖCK, Wilfried, 82152 Krailling (DE); WEINMAIER, Josef Helmut, 84453 Mühldorf (DE); ZELLNER, Helmut, 82278 Hörbach (DE)
(74) Vertreter: Dey, Michael
(86) Internationale Anmeldenummer: PCT/EP2007/056006
(87) Internationale Veröffentlichungsnummer: WO 2008/006666

(56) Entgegenhaltungen:
- WO-A-2007/042313
- DD-A1- 128 663
- DE-A1- 2 256 255
- DE-A1- 19 911 024
- JOHN C. MIDDLETON, KEITH J. CARPENTER: "Ullmann's Encyclopedia, Stirred-Tank and Loop Reactors" [Online] 15. Juni 2005 (2005-06-15), WILEY-VCH VERLAG GMBH & CO KGAA , WEINHEIM , XP002449394 Gefunden im Internet: URL:http://www.mrw.interscience.wiley.com/ emrw/9783527306732/ueic/article/b04_167/cu rrent/pdf> Seite 6 - Seite 9 Seite 6, Spalte 2 Seite 8, Spalte 1 Seite 8, Spalte 2

## Beschreibung

Die Erfindung richtet sich auf ein Verfahren zur Herstellung von Acylperoxiden, das sicher und mit hohen Raum-Zeit-Ausbeuten durchgeführt werden kann.

Acylperoxide werden üblicherweise durch Umsetzung einer Acylverbindung aus der Reihe der Carbonsäurechloride, Carbonsäureanhydride und Chlorformiate mit einer Persauerstoffverbindung aus der Reihe der organischen Hydroperoxide und Wasserstoffperoxid hergestellt. Die Umsetzung erfolgt unter Zusatz einer wässrigen Lösung eines Alkalimetallhydroxids oder Erdalkalimetallhydroxids, um die bei der Umsetzung der Acylverbindung mit der Persauerstoffverbindung freiwerdende Säure zu binden. Die Umsetzung erfolgt dabei in einer zweiphasigen Reaktionsmischung und verläuft exotherm.

Acylperoxide finden technische Anwendung als Initiatoren für Polymerisationsreaktionen, für Vernetzungsreaktionen von Polymeren und für die Härtung von ungesättigten Polyesterharzen. Diese Anwendungen basieren auf dem Zerfall der Acylperoxide an der labilen Sauerstoff-Sauerstoff Bindung in freie Radikale. Dieser Zerfall erfolgt in Abhängigkeit der chemischen Struktur des Peroxids bei unterschiedlichen Temperaturen und mit unterschiedlicher Geschwindigkeit. Die beim Zerfall freigesetzte Wärme führt im Fall einer ungenügenden Wärmeabfuhr zu einer Selbstbeschleunigung des Zerfallsvorgangs, der in eine zumeist heftige Zersetzung mündet und im schlimmsten Fall zur Explosion führen kann.

Für die sichere Herstellung von Acylperoxiden werden deshalb Verfahren benötigt, die sich unterhalb der Temperatur der selbstbeschleunigenden Zersetzung (SADT) durchführen lassen und die mit geringem Reaktorvolumen und hoher Raum-Zeit-Ausbeute ablaufen, so dass im Reaktor jeweils nur eine geringe Menge an Acylperoxid vorliegt.

US 3,849,468 offenbart ein kontinuierliches Verfahren zur Herstellung von Acylperoxiden in einem Schlaufenreaktor. Bei diesem Verfahren wird nur die wässrige Phase der Reaktionsmischung im Kreis geführt, da nach jedem Durchlauf durch die Reaktionssäule die organische Phase mit dem Produkt abgetrennt und nur die wässrige Phase in die Reaktionssäule zurückgeführt wird. Das Verfahren erfordert die Verwendung eines Lösungsmittels und ermöglicht Räum-Zeit-Ausbeuten im Bereich von 0,1 bis 0,47 mol/l h, wie aus den Beispielen hervorgeht.

US 4,075,236 offenbart ein demgegenüber verbessertes Verfahren, bei dem die Umsetzung kontinuierlich in zwei in Reihe geschalteten Rührkesseln erfolgt, wobei die Einsatzstoffe dem ersten Rührkessel zugeführt werden. Das Verfahren ermöglicht eine Reaktionsführung ohne Lösungsmittel mit Raum-Zeit-Ausbeuten im Bereich von 1,3 bis 2,0 mol/l·h.

DD 128 663 offenbart ein zu US 4,075,236 analoges Verfahren, bei dem die Umsetzung kontinuierlich in 4 bis 7 in Reihe geschalteten Rührkesseln erfolgt. Bei Reaktionstemperaturen von 40 bis 95°C, die oberhalb der Temperatur der selbstbeschleunigenden Zersetzung (SADT) liegen, werden Raum-Zeit-Ausbeuten im Bereich von 16 bis 74 mol/l·h erzielt.

EP 847 387 beschreibt ein Verfahren zur kontinuierlichen Herstellung von Acylperoxiden in einer Durchlaufapparatur, die einen Mischreaktor und eine nachgeschaltete Verbindungsleitung umfasst, wobei mindestens 10% des Umsatzes in der nachgeschalteten Verbindungsleitung erfolgen. In dem Mischreaktor wird ein Intensivmischer in Form eines Strahlmischers, Ultraschallerzeugers, statischen Mischers oder Rotor/Stator-Mischers verwendet. Das beschriebene Verfahren lässt sich nicht sicher in einen technischen Maßstab übertragen, da bei mindestens 10% des Umsatzes in der nachgeschalteten Verbindungsleitung die erforderliche Wärmeabfuhr nur bei den kleinen Leitungsquerschnitten einer Laborapparatur sichergestellt werden kann.

US 2004/0249097 beschreibt ein Verfahren zur Herstellung von Emulsionen von Acylperoxiden, die Tropfengrößen des Acylperoxids von weniger als 10 µm aufweisen. Bei dem Verfahren werden eine Acylverbindung und eine Persauerstoffverbindung in Gegenwart eines Dispergiermittels umgesetzt. In einer Ausführungsform des Verfahrens erfolgt die Umsetzung in einem
Durchflusshomogenisator (in-line homogenizer), der mit einer Kreislaufführung betrieben werden kann, um mehrere Durchläufe für die Homogenisierung zu erreichen. Mit diesem Verfahren lassen sich allerdings nur Emulsionen von Acylperoxiden erhalten und nicht Acylperoxide in reiner Form.

Es wurde nun gefunden, dass sich die Aufgabe einer sicheren Herstellung von Acylperoxiden bei niederen Temperaturen und hohen Raum-Zeit-Ausbeuten durch dass erfindungsgemäße Verfahren lösen lässt, das außerdem die Herstellung von Acylperoxiden in reiner Form ohne die Verwendung eines Lösungsmittels ermöglicht.

Gegenstand der Erfindung ist ein kontinuierliches Verfahren zur Herstellung von Acylperoxiden durch Umsetzung einer Acylverbindung aus der Reihe der Carbonsäurechloride, Carbonsäureanhydride und Chlorformiate mit einer Persauerstoffverbindung aus der Reihe der organischen Hydroperoxide und Wasserstoffperoxid, wobei die Umsetzung in mindestens zwei in Reihe geschalteten durchmischten Reaktionszonen erfolgt und der ersten Reaktionszone die Acylverbindung, die Persauerstoffverbindung und eine wässrige Lösung einer Base zugeführt werden, wobei das Verfahren dadurch gekennzeichnet ist, dass die erste Reaktionszone einen Kreislauf der zweiphasigen Reaktionsmischung über einen Wärmetauscher umfasst, in dem die Reaktionsmischung gekühlt wird.

Bei dem erfindungsgemäßen Verfahren werden eine Acylverbindung und eine Persauerstoffverbindung in mindestens zwei in Reihe geschalteten durchmischten Reaktionszonen umgesetzt. Durchmischte Reaktionszonen im Sinne der Erfindung sind dabei Reaktionszonen, in denen durch die Vermischung eine weitgehend einheitliche Zusammensetzung der Reaktionsmischung innerhalb der Reaktionszone gewährleistet ist. Zwischen den in Reihe geschalteten Reaktionszonen findet dagegen keine Rückvermischung statt, das heißt von einer nachgeschalteten Reaktionszone wird keine Reaktionsmischung in eine vorangehende Reaktionszone zurückgeführt. Die durchmischten Reaktionszonen können als separate Reaktoren ausgeführt sein. Alternativ können auch zwei oder mehr durchmischte Reaktionszonen in einem gemeinsamen Apparat ausgeführt sein. Geeignete Ausführungsformen für durchmischte Reaktionszonen sind beispielsweise Rührkesselreaktoren oder Schlaufenreaktoren. Vorzugsweise umfasst das erfindungsgemäße Verfahren 2 bis 8 in Reihe geschaltete durchmischte Reaktionszonen.

Bei dem erfindungsgemäßen Verfahren werden die Acylverbindung und die Persauerstoffverbindung der ersten Reaktionszone zugeführt. Der ersten Reaktionszone wird außerdem eine wässrige Lösung einer Base zugeführt. Als Base können wasserlöslich Metallhydroxide, wasserlösliche quartäre Ammoniumhydroxide oder wasserlösliche tertiäre Amine verwendet werden. Bevorzugt wird als Base ein Alkalimetallhydroxid oder ein Erdalkalimetallhydroxid verwendet, besonders bevorzugt Natriumhydroxid oder Kaliumhydroxid. Die Persauerstoffverbindung und die wässrige Lösung der Base können gemischt werden, bevor sie der ersten Reaktionszone zugeführt werden. Alternativ können sie der ersten Reaktionszone auch getrennt zugeführt werden. Die Zuführung von Acylverbindung und Persauerstoffverbindung kann kontinuierlich oder intermittierend erfolgen, wobei eine kontinuierliche Zuführung bevorzugt wird. Auch die wässrige Lösung der Base kann wahlweise kontinuierlich oder intermittierend zugeführt werden. In einer bevorzugten Ausführungsform wird die Zufuhr der Lösung der Base über eine Messung des pH-Werts in der wässrigen Phase in der ersten Reaktionszone überwacht.

In der ersten Reaktionszone bildet sich bei dem erfindungsgemäßen Verfahren eine zweiphasige Reaktionsmischung aus, die zwei flüssige Phasen aufweist. Die erste, wässrige Phase enthält den größten Teil der eingesetzten Base und des bei der Reaktion mit der Base gebildeten Chloridsalzes, beziehungsweise Carboxylatsalzes. Die zweite, organische Phase enthält den größten Teil der eingesetzten Acylverbindung und des bei der Reaktion entstehenden Acylperoxids.

Bei dem erfindungsgemäßen Verfahren wird in der ersten Reaktionszone die zweiphasige Reaktionsmischung in einem Kreislauf geführt, der einen Wärmetauscher umfasst, in dem die Reaktionsmischung gekühlt wird. Vorzugsweise wird bei jedem Durchlaufen des Kreislaufs die Reaktionsmischung ganz oder teilweise über den Wärmetauscher geführt. Besonders bevorzugt wird bei jedem Durchlaufen des Kreislaufs die gesamte Reaktionsmischung über den Wärmetauscher geführt. In einer besonders bevorzugten Ausführungsform wird als erste Reaktionszone ein Schlaufenreaktor verwendet, in dem die Reaktionsmischung mit einer Pumpe durch einen Wärmetauscher gefördert wird.

Die Geschwindigkeit des Kreislaufs in der ersten Reaktionszone wird vorzugsweise so gewählt, dass sich ein Kreislaufverhältnis von mindestens 2, vorzugsweise mindestens 5 ergibt. Das Kreislaufverhältnis beträgt aus wirtschaftlichen Gründen in der Regel weniger als 1000, insbesondere weniger als 100. Das Kreislaufverhältnis steht dabei für das Massenverhältnis von in der ersten Reaktionszone umlaufender Reaktionsmischung zu der ersten Reaktionszone entnommener Reaktionsmischung.

Als Wärmetauscher können alle aus dem Stand der Technik bekannten Wärmetauscher verwendet werden, die sich zur Kühlung einer zweiphasigen flüssigen Mischung eignen. Vorzugsweise werden Rohrbündelwärmetauscher oder Plattenwärmetauscher verwendet, bei denen die Reaktionsmischung durch die Rohre beziehungsweise Spalte zwischen den Platten strömt. Der Wärmetauscher wird vorzugsweise so ausgeführt, dass die Reaktionsmischung durch Kanäle oder Spalte des Wärmetauschers gefördert wird, die einen hydraulischen Durchmesser von weniger als 50 mm, besonders bevorzugt weniger als 10 mm aufweisen. Der Wärmetauscher wird so dimensioniert, dass das Verhältnis zwischen der Kühlfläche des Wärmetauschers und dem Volumen der ersten Reaktionszone mindestens 1,5 m²/m³ und besonders bevorzugt mindestens 15 m²/m³ beträgt. Vorzugsweise werden Bauweise und Dimensionierung des Wärmetauschers so gewählt, dass sich innerhalb des Wärmetauschers eine überwiegend turbulente Strömung einstellt, die in der zweiphasigen Reaktionsmischung eine Dispergierung durch Tropfenzerkleinerung bewirkt.

Die Zugabe der Acylverbindung in die erste Reaktionszone erfolgt vorzugsweise in den Kreislauf der ersten Reaktionszone, besonders bevorzugt an einer Stelle, an der die Strömungsgeschwindigkeit der Reaktionsmischung mehr als 0,05 m/s, insbesondere mehr als 0,5 m/s beträgt.

In einer bevorzugten Ausführungsform des erfindungsgemäßen Verfahrens umfasst der Kreislauf in der ersten Reaktionszone auch einen Mischer, der in der zweiphasigen Reaktionsmischung eine Dispergierung durch Tropfenzerkleinerung bewirkt. In dieser Ausführungsform erfolgt die Zugabe der Acylverbindung in die erste Reaktionszone vorzugsweise unmittelbar vor dem Mischer. Als Mischer eignen sich alle Mischer, die dem Fachmann als geeignet für die Tropfenzerkleinerung in zweiphasigen flüssigen Mischungen bekannt sind. Besonders geeignet sind statische Mischer, beispielsweise statische Mischer vom Bautyp der Sulzer SMV- oder SMX-Mischer. In einer weiteren bevorzugten Ausführungsform wird als Mischer eine Umwälzpumpe für den Kreislauf in der ersten Reaktionszone verwendet, insbesondere eine Kreiselpumpe. Bei geeigneter Bauweise und Dimensionierung eignet sich auch, wie oben beschrieben, der im erfindungsgemäßen Verfahren verwendete Wärmetauscher als Mischer.

In einer weiteren bevorzugten Ausführungsform des erfindungsgemäßen Verfahrens sind die der ersten Reaktionszone nachgeschalteten Reaktionszonen in Form eines Rührzellenreaktors ausgeführt. Ein Rührzellenreaktor weist in einem Gehäuse zwei oder mehr in Reihe geschaltete Reaktionszonen auf, wobei jede der Reaktionszonen durch mindestens einen Rührer durchmischt wird und die Rührer von einem gemeinsamen Motor angetrieben werden, vorzugsweise über eine gemeinsame Antriebswelle. Die Reaktionszonen sind dabei so miteinander verbunden, dass praktisch keine Rückvermischung der Reaktionsmischung von einer Reaktionszone in die vorangehende Reaktionszone erfolgt. Die Reaktionszonen können beispielsweise voneinander getrennt und nur über Rohrleitungen miteinander verbunden sein. Alternativ können die Reaktionszonen aber auch über Siebböden oder Ventilböden miteinander verbunden sein, die so dimensioniert sind, dass praktisch keine Rückvermischung erfolgt.

Die der ersten Reaktionszone nachgeschalteten Reaktionszonen können Kühleinrichtungen aufweisen, mit denen die in diesen Reaktionszonen freigesetzte Reaktionswärme abgeführt wird. Als Kühleinrichtungen können beispielsweise Wärmetauscher wie in der ersten Reaktionszone verwendet werden. Alternativ können auch Reaktoren mit Kühlmantel verwendet werden.

Im erfindungsgemäßen Verfahren wird eine Acylverbindung aus der Reihe der Carbonsäurechloride, Carbonsäureanhydride und Chlorformiate eingesetzt.

Als Carbonsäurechlorid wird vorzugsweise eine Verbindung aus der Reihe Acetylchlorid, Propionylchlorid, Butyrylchlorid, Isobutyrylchlorid, Valeroylchlorid, 2-Methylbutyrylchlorid, Pivaloylchlorid, 2-Methylpentanoylchlorid, 2-Ethylbutyrylchlorid, 2-Ethylhexanoylchlorid, Nonanoylchlorid, 2,4,4-Trimethylpentanoylchlorid, 3,5,5-Trimethylhexanoylchlorid, Decanoylchlorid, Neodecanoylchlorid, Lauroylchlorid, Benzoylchlorid, 2-Methylbenzoylchlorid, 4-Methylbenzoylchlorid, 4-Chlorbenzoylchlorid, 2,4-Dichlorbenzoylchlorid und Naphthoylchlorid eingesetzt. Besonders bevorzugt wird als Carbonsäurechlorid Pivaloylchlorid, 2-Ethylhexanoylchlorid oder Benzoylchlorid verwendet.

Als Carbonsäureanhydrid wird vorzugsweise eine Verbindung aus der Reihe Acetanhydrid, Bernsteinsäureanhydrid, Maleinsäureanhydrid und Phthalsäureanhydrid eingesetzt. Als Chlorformiat wird vorzugsweise eine Verbindung aus der Reihe Methylchlorformiat, Ethylchlorformiat, n-Propylchlorformiat, Isopropylchlorformiat, n-Butylchlorformiat, sec-Butylchlorformiat, 2-Ethylhexylchlorformiat, Isotridecylchlorformiat, Myristylchlorformiat, Cetylchlorformiat, Stearylchlorformiat, Cyclohexylchlorformiat, 4-tert-Butylcyclohexylchlorformiat, Benzylchlorformiat und 2-Phenoxyethylchlorformiat eingesetzt. Besonders bevorzugt wird als Chlorformiat 2-Ethylhexylchlorformiat verwendet.

Im erfindungsgemäßen Verfahren wird eine Persauerstoffverbindung aus der Reihe der organischen Hydroperoxide und Wasserstoffperoxid eingesetzt.

Als organisches Hydroperoxid wird vorzugsweise eine Verbindung aus der Reihe tert-Butylhydroperoxid, tert-Amylhydroperoxid, 1,1,3,3-Tetramethylbutylhydroperoxid, 2,5-Dimethylhexan-2,5-dihydroperoxid, 2,5-Dimethyl-3-hexin-2,5-dihydroperoxid, p-Menthanhydroperoxid, Pinanhydroperoxid, Tetralinhydroperoxid, Cumolhydroperoxid, 4-tert-Butylcumolhydroperoxid, 1,3-Diisopropylbenzoldihydroperoxid und
1,4-Diisopropylbenzoldihydroperoxid eingesetzt. Besonders bevorzugt wird als organisches Hydroperoxid tert-Butylhydroperoxid verwendet.

Acylverbindungen und/oder organische Hydroperoxide, die unter den verwendeten Reaktionsbedingungen fest sind, werden vorzugsweise in Form einer Lösung in einem Lösungsmittel eingesetzt. Als Lösungsmittel eignen sich dabei alle dem Fachmann bekannten Lösungsmittel, die unter den Reaktionsbedingungen weder mit der Acylverbindung, noch mit dem organischen Hydroperoxid oder der Base reagieren. Vorzugsweise werden Lösungsmittel eingesetzt, die bei der verwendeten Reaktionstemperatur eine Löslichkeit in Wasser von weniger als 1 g/l aufweisen. Geeignete Lösungsmittel sind zum Beispiel Toluol und Isododecan.

Bei der Herstellung von Acylperoxiden, die unter den verwendeten Reaktionsbedingungen fest sind, wird vorzugsweise ein Lösungsmittel für das Acylperoxid in einer Menge zugesetzt, die nach der letzten Reaktionszone zu einer zweiphasigen Reaktionsmischung führt, die als organische Phase eine flüssige Lösung des Acylperoxids in dem Lösungsmittel aufweist. Vorzugsweise werden Lösungsmittel eingesetzt, die bei der verwendeten Reaktionstemperatur eine Löslichkeit in Wasser von weniger als 1 g/l aufweisen. Geeignete Lösungsmittel sind zum Beispiel Toluol und Isododecan.

Bei der Herstellung von Acylperoxiden, die unter den verwendeten Reaktionsbedingungen flüssig sind, wird die Umsetzung vorzugsweise ohne Lösungsmittel durchgeführt.

Bei der Umsetzung eines Carbonsäurechlorids der Struktur R¹C(O)C1 oder eines Carbonsäureanhydrids der Struktur R¹C(O)OC(O)R¹ mit einem Hydroperoxid der Struktur R²OOH wird in dem erfindungsgemäßen Verfahren ein Percarbonsäureester der Struktur R¹C(O)OOR² erhalten. Die Acylverbindung und das Hydroperoxid werden dabei vorzugsweise in einem Molverhältnis von 1 : 0,8 bis 1 : 2, besonders bevorzugt 1 : 1 bis 1 : 1,5 eingesetzt. Das Molverhältnis von Acylverbindung zu Base (bevorzugt als Alkalimetallhydroxid) liegt vorzugsweise im Bereich von 1 : 0,8 bis 1 : 5, besonders bevorzugt 1 : 1 bis 1 : 3,5. Die Zugabe der Base erfolgt vorzugsweise so, dass sich in der ersten Reaktionszone in der wässrigen Phase der Reaktionsmischung ein pH-Wert im Bereich von 8 bis 14, vorzugsweise 11 bis 14 ergibt. Die Umsetzung erfolgt vorzugsweise bei einer Temperatur im Bereich von -10 bis 50 °C, besonders bevorzugt 10 bis 40 °C.

Bei der Umsetzung eines Carbonsäurechlorids der Struktur R¹C(O)Cl oder eines Carbonsäureanhydrids der Struktur R¹C(O)OC(O)R¹ mit Wasserstoffperoxid wird in dem erfindungsgemäßen Verfahren ein Diacylperoxid der Struktur R¹C(O)OOC(O)R¹ erhalten. Die Acylverbindung und Wasserstoffperoxid werden dabei vorzugsweise in einem Molverhältnis von 2 : 1 bis 2 : 4, besonders bevorzugt 2 : 1 bis 2 : 1,5 eingesetzt. Das Molverhältnis von Acylverbindung zu Base (bevorzugt als Alkalimetallhydroxid) liegt vorzugsweise im Bereich von 1 : 1 bis 1 : 2, besonders bevorzugt 1 : 1 bis 1 : 1,5. Die Zugabe der Base erfolgt vorzugsweise so, dass sich in der ersten Reaktionszone in der wässrigen Phase der Reaktionsmischung ein pH-Wert im Bereich von 8 bis 14, vorzugsweise 10 bis 14 ergibt. Die Umsetzung erfolgt vorzugsweise bei einer Temperatur im Bereich von -10 bis 50 °C, besonders bevorzugt 0 bis 40 °C.

Bei der Umsetzung eines Chlorformiats der Struktur R¹OC(O)Cl mit einem Hydroperoxid der Struktur R²OOH wird in dem erfindungsgemäßen Verfahren ein Peroxocarbonat der Struktur R¹OC(O)OOR² erhalten. Das Chlorformiat und das Hydroperoxid werden dabei vorzugsweise in einem Molverhältnis von 1 : 0,8 bis 1 : 2, besonders bevorzugt 1 : 1,2 bis 1 : 1,5 eingesetzt. Das Molverhältnis von Chlorformiat zu Base (bevorzugt als Alkalimetallhydroxid) liegt vorzugsweise im Bereich von 1 : 0,8 bis 1 : 2, besonders bevorzugt 1 : 1,2 bis 1 : 1,5. Die Zugabe der Base erfolgt vorzugsweise so, dass sich in der ersten Reaktionszone in der wässrigen Phase der Reaktionsmischung ein pH-Wert im Bereich von 8 bis 14, vorzugsweise 10 bis 14 ergibt. Die Umsetzung erfolgt vorzugsweise bei einer Temperatur im Bereich von -10 bis 50 °C, besonders bevorzugt 0 bis 40 °C.

Bei der Umsetzung eines Chlorformiats der Struktur R¹OC(O)Cl mit Wasserstoffperoxid wird in dem erfindungsgemäßen Verfahren ein Peroxodicarbonat der Struktur R¹OC(O)OOC(O)OR¹ erhalten. Das Chlorformiat und Wasserstoffperoxid werden dabei vorzugsweise in einem Molverhältnis von 2 : 1 bis 2 : 4, besonders bevorzugt 2 : 1 bis 2 : 1,5 eingesetzt. Das Molverhältnis von Chlorformiat zu Base (bevorzugt als Alkalimetallhydroxid) liegt vorzugsweise im Bereich von 1 : 1 bis 1 : 2, besonders bevorzugt 1 : 1 bis 1 : 1,5. Die Zugabe der Base erfolgt vorzugsweise so, dass sich in der ersten Reaktionszone in der wässrigen Phase der Reaktionsmischung ein pH-Wert im Bereich von 8 bis 14, vorzugsweise 10 bis 14 ergibt. Die Umsetzung erfolgt vorzugsweise bei einer Temperatur im Bereich von -10 bis 50 °C, besonders bevorzugt 0 bis 40 °C.

Die Mengen an Acylverbindung, Persauerstoffverbindung und wässriger Lösung von Base, die der ersten Reaktionszone zugeführt werden, werden vorzugsweise so gewählt, dass in der ersten Reaktionszone ein Umsatz an Acylverbindung im Bereich von 60 bis 99 %, besonders bevorzugt 85 bis 95 %, erreicht wird.

Der letzten Reaktionszone wird eine zweiphasige Produktmischung entnommen, die das hergestellte Acylperoxid in der organischen Phase enthält. Diese Produktmischung kann nach bekannten Verfahren weiter aufgearbeitet und gereinigt werden. Vorzugsweise wird die organische Phase mit dem Acylperoxid durch eine Phasentrennung abgetrennt und anschließend einer Extraktion mit einer alkalischen wässrigen Lösung unterzogen, um nicht umgesetzte Persauerstoffverbindung und bei der Reaktion gebildete Nebenprodukte, wie zum Beispiel durch Hydrolyse der Acylverbindung gebildete Carbonsäure, zu entfernen. Das Acylperoxid kann anschließend weiteren Wäschen und einer Trocknung unterzogen werden, wobei dazu geeignete Verfahren dem Fachmann aus US 4,075,236 bekannt sind.

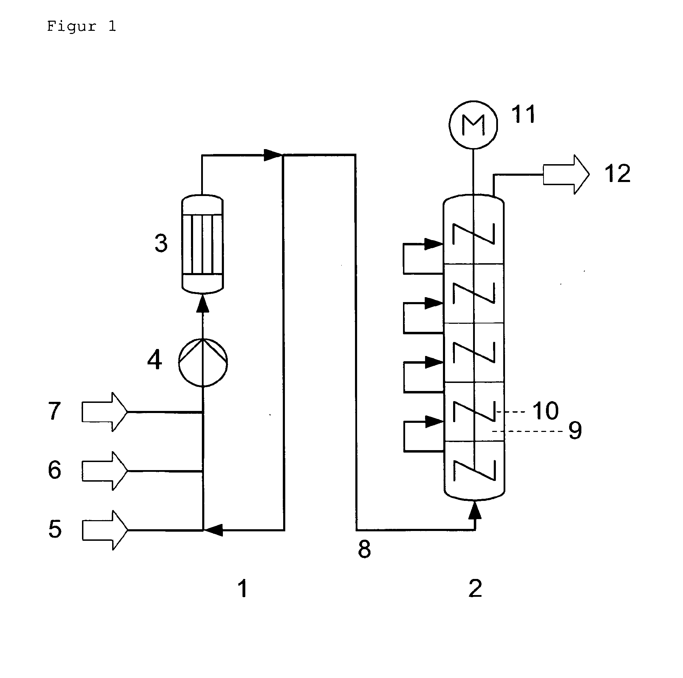

Figur 1 zeigt eine bevorzugte Ausführungsform des erfindungsgemäßen Verfahrens, bei dem die Umsetzung in 6 in Reihe geschalteten Reaktionszonen erfolgt. Die erste Reaktionszone ist dabei als Schlaufenreaktor (1) ausgeführt, die zweite bis sechste Reaktionszone sind als Rührzellenreaktor (2) ausgeführt. Der Schlaufenreaktor (1) umfasst einen Wärmetauscher (3) in Form eines Plattenwärmetauschers und eine Pumpe (4) in Form einer Kreiselpumpe, die zugleich auch als Mischer für die zweiphasige Reaktionsmischung wirkt. Dem Schlaufenreaktor (1) werden die Persauerstoffverbindung (5), eine wässrige Lösung einer Base (6) und die Acylverbindung (7) zugeführt, wobei die Zugabe der Acylverbindung unmittelbar vor der als Mischer wirkenden Pumpe (4) erfolgt. Aus dem Schlaufenreaktor (1) wird über eine Verbindungsleitung (8) Reaktionsmischung dem Rührzellenreaktor (2) zugeführt. In dem Rührzellenreaktor (2) durchläuft die Reaktionsmischung von unten nach oben 5 Reaktionszonen (9), die jeweils von einem Rührer (10) durchmischt werden, wobei alle Rührer von einem Motor (11) über eine gemeinsame Welle angetrieben werden. Der letzten Reaktionszone wird eine zweiphasige Produktmischung (12) entnommen, die das hergestellte Acylperoxid in der organischen Phase enthält.

Das erfindungsgemäße Verfahren ermöglicht die Herstellung von Acylperoxiden mit verbesserten Raum-Zeit-Ausbeuten und höheren Produktselektivitäten. Das Verfahren lässt sich auch ohne Lösungsmittel sicher durchführen, da die Umsetzung bei Temperaturen unterhalb der selbstbeschleunigenden Zersetzungstemperatur des Acylperoxids durchgeführt werden kann und sich die bei der Reaktion freiwerdende Reaktionswärme zuverlässig abführen lässt. Mit dem erfindungsgemäßen Verfahren lässt sich das für eine gewünschte Produktionskapazität erforderliche Reaktorvolumen verkleinern, so dass Risiken durch Betriebsstörungen verringert werden.

Die folgenden Beispiele illustrieren das erfindungsgemäße Verfahren, ohne es jedoch zu beschränken.

### Beispiele

Verwendete Reaktoren:
Reaktor Typ A:
   Rührzellenreaktor mit 7 gleich großen, übereinander angeordneten Kammern mit einem gemeinsamen Kühlmantel, die durch 6 gekühlte Böden voneinander getrennt und über Durchlässe zwischen benachbarten Kammern miteinander verbunden sind. In jeder Kammer ist ein Rührer angebracht, wobei alle Rührer über eine gemeinsame Welle angetrieben werden. Die Einspeisung der Einsatzstoffe oder der Reaktionsmischung eines vorgeschalteten Reaktors erfolgt in die unterste Kammer. Die Entnahme der umgesetzten Reaktionsmischung erfolgt durch Überlauf aus der obersten Kammer.
Reaktor Typ B:
   Rührzellenreaktor mit gleichem Aufbau wie Reaktor A, aber dreifachem Volumen.
Reaktor Typ C:
   Rührzellenreaktor mit gleichem Aufbau wie Reaktor A, aber mit 6 Kammern von je 1,7 1 Volumen.
Reaktor Typ D:
   Rührkessel mit Kühlmantel und innenliegender Kühlschlange und 0,53-fachem Nutzvolumen von Reaktor B.
Reaktor Typ E:
   Schlaufenreaktor mit einem Gesamtvolumen von 2,2 l, bestehend aus einem Plattenwärmetauscher mit einem Plattenabstand von 1 mm und einer Wärmetauscherfläche von 0,7 m², einer Umwälzpumpe mit einer Förderleistung von 1 m³/h, sowie Verbindungsleitungen zwischen Wärmetauscher und Umwälzpumpe mit einem Innendurchmesser von 14 mm. Die Einspeisung der Einsatzstoffe erfolgt in die Verbindungsleitung unmittelbar vor der Umwälzpumpe. Der Reaktor weist ein Verhältnis von Kühlfläche des Wärmetauschers zum Volumen der Reaktionszone von 318 m²/m³ auf. Die Strömungsgeschwindigkeit an der Zugabestelle der Einsatzstoffe liegt bei 1,8 m/s.

### Beispiel 1 (nicht erfindungsgemäß)

### Herstellung von tert-Butylperoxy-2-ethylhexanoat

Die Umsetzung erfolgt in einer Reaktoranordnung aus einem ersten Reaktor vom Typ A mit einem nachgeschalteten Reaktor vom Typ B. Dem ersten Reaktor werden 2,42 kg/l·h einer 26 Gew.-% Lösung von tert-Butylhydroperoxid und 17 Gew.-% Kaliumhydroxid in Wasser und 0,86 kg/l·h 2-Ethylhexanoylchlorid zugeführt. Die Dosierraten beziehen sich dabei auf das Gesamtvolumen der Reaktoranordnung in Litern. Durch Kühlen mit Kühlwasser wird die Innentemperatur in beiden Reaktoren auf 46 °C gehalten. Die dem zweiten Reaktor entnommene Reaktionsmischung wird mit 1,5 kg/l·h entsalztem Wasser gemischt und die organische Phase wird abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit entsalztem Wasser gewaschen und danach mit Calciumchlorid getrocknet. Man erhält 1,06 kg/l·h tert-Butylperoxy-2-ethylhexanoat (93,1 % bezogen auf 2-Ethylhexanoylchlorid).

### Beispiel 2 (erfindungsgemäß)

### Herstellung von tert-Butylperoxy-2-ethylhexanoat

Die Umsetzung erfolgt in einer Reaktoranordnung aus einem ersten Reaktor vom Typ E mit einem nachgeschalteten Reaktor vom Typ C. Der erste Reaktor wird vor Reaktionsbeginn mit einer Lösung von 25,8 Gew.-% tert-Butylhydroperoxid und 16,4 Gew.-% Kaliumhydroxid in Wasser gefüllt. Dann werden dem ersten Reaktor 24,6 kg/h einer Lösung von 70 Gew.-% tert-Butylhydroperoxid in Wasser, 24,2 kg/h einer Lösung von 45 Gew.-% Kaliumhydroxid in Wasser, 17,4 kg/h Wasser und 24,0 kg/h 2-Ethylhexanoylchlorid zugeführt. Durch Kühlen mit Kühlwasser wird die Innentemperatur im ersten Reaktor auf 35 °C und im zweiten Reaktor auf 27 °C gehalten. Aus der dem zweiten Reaktor entnommenen Reaktionsmischung wird die organische Phase abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit einer 1 Gew.-% Lösung von Natriumhydrogencarbonat in Wasser gewaschen und danach durch Strippen in einer Füllkörperkolonne bei 33 °C und 35 bis 40 mbar getrocknet. Man erhält 31,4 kg/h tert-Butylperoxy-2-ethylhexanoat (98,5 % bezogen auf 2-Ethylhexanoylchlorid). Die Raum-Zeit-Ausbeute liegt bei 2,53 kg/l·h.

### Beispiel 3 (nicht erfindungsgemäß)

### Herstellung von tert-Amylperoxypivalat

Die Umsetzung erfolgt in einer Reaktoranordnung aus einem ersten Reaktor vom Typ D mit zwei in Reihe nachgeschalteten Reaktoren vom Typ B. Dem ersten Reaktor werden 0,26 kg/l·h einer 88 Gew.-% Lösung von tert-Amylhydroperoxid in Wasser, 0,47 kg/l·h einer 25 Gew.-% Lösung von Kaliumhydroxid in Wasser, 0,50 kg/l·h einer 25 Gew.-% Lösung von Natriumhydroxid in Wasser und 0,26 kg/l·h Pivaloylchlorid zugeführt. Die Dosierraten beziehen sich dabei auf das Gesamtvolumen der Reaktoranordnung in Litern. Durch Kühlen mit Kühlwasser wird die Innentemperatur im ersten Reaktor auf 25 °C und in den beiden nachfolgenden Reaktoren auf 15 °C gehalten. Die dem dritten Reaktor entnommene Reaktionsmischung wird mit 0,79 kg/l·h entsalztem Wasser und 0,08 kg/l·h Isododecan gemischt und die organische Phase wird abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit entsalztem Wasser gewaschen und danach mit Calciumchlorid getrocknet. Man erhält 0,42 kg/l·h einer 75 Gew.-% Lösung von tert-Amylperoxypivalat in Isododecan (80,6 % bezogen auf Pivaloylchlorid).

Beispiel 4 (erfindungsgemäß)

### Herstellung von tert-Amylperoxypivalat

Die Umsetzung erfolgt in einer Reaktoranordnung aus einem ersten Reaktor vom Typ E mit einem nachgeschalteten Reaktor vom Typ C. Der erste Reaktor wird vor Reaktionsbeginn mit einer Lösung von 23,1 Gew.-% tert-Amylhydroperoxid, 9,9 Gew.-% Kaliumhydroxid und 8,3 Gew.-% Natriumhydroxid in Wasser gefüllt. Dann werden dem ersten Reaktor 20,2 kg/h einer Lösung von 88 Gew.-% tert-Amylhydroperoxid in Wasser, 15,8 kg/h einer Lösung von 45 Gew.-% Kaliumhydroxid in Wasser, 12,2 kg/h einer Lösung von 50 Gew.-% Natriumhydroxid in Wasser, 24,4 kg/h Wasser und 18,0 kg/h Pivaloylchlorid zugeführt. Durch Kühlen mit Kühlwasser wird die Innentemperatur im ersten Reaktor auf 18 °C und im zweiten Reaktor auf 10 °C gehalten. Die dem zweiten Reaktor entnommene Reaktionsmischung wird mit 8,8 kg/h Isododecan gemischt und die organische Phase wird abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit einer 1 Gew.-% Lösung von Natriumhydrogencarbonat in Wasser gewaschen und danach durch Strippen in einer Füllkörperkolonne bei 20 °C und 45 mbar getrocknet. Man erhält 35,0 kg/h einer 75 Gew.-% Lösung von tert-Amylperoxypivalat in Isododecan (93,5 % bezogen auf Pivaloylchlorid). Die Raum-Zeit-Ausbeute liegt bei 2,82 kg/l·h.

### Beispiel 5 (nicht erfindungsgemäß)

### Herstellung von tert-Butylperoxy-3,5,5-trimethylhexanoat

Die Umsetzung erfolgt in einer Reaktoranordnung aus einem ersten Reaktor vom Typ A mit einem nachgeschalteten Reaktor vom Typ B. Dem ersten Reaktor werden 2,25 kg/l·h einer 26 Gew.-% Lösung von tert-Butylhydroperoxid und 17 Gew.-% Kaliumhydroxid in Wasser und 0,75 kg/l·h
3,5,5-Trimethylhexanoylchlorid zugeführt. Die Dosierraten beziehen sich dabei auf das Gesamtvolumen der Reaktoranordnung in Litern. Durch Kühlen mit Kühlwasser wird die Innentemperatur in beiden Reaktoren auf 46 °C gehalten. Die dem zweiten Reaktor entnommene Reaktionsmischung wird mit 1,5 kg/l·h entsalztem Wasser gemischt und die organische Phase wird abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit entsalztem Wasser gewaschen und danach mit Calciumchlorid getrocknet. Man erhält 0,90 kg/l·h tert-Butylperoxy-3,5,5-Trimethylhexanoat (91,6 % bezogen auf 3,5,5-Trimethylhexanoylchlorid).

### Beispiel 6 (erfindungsgemäß)

### Herstellung von tert-Butylperoxy-3,5,5-trimethylhexanoat

Die Umsetzung erfolgt in einer Reaktoranordnung aus einem ersten Reaktor vom Typ E mit einem nachgeschalteten Reaktor vom Typ C. Der erste Reaktor wird vor Reaktionsbeginn mit einer Lösung von 25,6 Gew.-% tert-Butylhydroperoxid und 15,0 Gew.-% Kaliumhydroxid in Wasser gefüllt. Dann werden dem ersten Reaktor 20,2 kg/h einer Lösung von 70 Gew.-% tert-Butylhydroperoxid in Wasser, 18,2 kg/h einer Lösung von 45 Gew.-% Kaliumhydroxid in Wasser, 16,2 kg/h Wasser und 19,2 kg/h 3,5,5-Trimethylhexanoylchlorid zugeführt. Durch Kühlen mit Kühlwasser wird die Innentemperatur im ersten Reaktor auf 25 °C und im zweiten Reaktor auf 25 °C gehalten. Aus der dem zweiten Reaktor entnommenen Reaktionsmischung wird die organische Phase abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit einer 1 Gew.-% Lösung von Natriumhydrogencarbonat in Wasser gewaschen und danach durch Strippen in einer Füllkörperkolonne bei 34 °C und 47 mbar getrocknet. Man erhält 24,6 kg/h tert-Butylperoxy-3,5,5-trimethylhexanoat (98,2 % bezogen auf 3,5,5-Trimethylhexanoylchlorid). Die Raum-Zeit-Ausbeute liegt bei 1,98 kg/l·h.

Beispiel 7 (nicht erfindungsgemäß)

### Herstellung von tert-Butylperoxyisobutyrat

Die Umsetzung erfolgt in einer Reaktoranordnung aus einem ersten Reaktor vom Typ D mit zwei in Reihe nachgeschalteten Reaktoren vom Typ B. Dem ersten Reaktor werden 1,22 kg/l·h einer 26 Gew.-% Lösung von tert-Butylhydroperoxid und 17 Gew.-% Kaliumhydroxid in Wasser und 0,32 kg/l·h Isobutyroylchlorid zugeführt. Die Dosierraten beziehen sich dabei auf das Gesamtvolumen der Reaktoranordnung in Litern. Durch Kühlen mit Kühlwasser wird die Innentemperatur im ersten Reaktor auf 26 °C und in den beiden nachfolgenden Reaktoren auf 20 °C gehalten. Die dem dritten Reaktor entnommene Reaktionsmischung wird mit 0,79 kg/l·h entsalztem Wasser und 0,14 kg/l·h Isododecan gemischt und die organische Phase wird abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit entsalztem Wasser gewaschen und danach mit Calciumchlorid getrocknet. Man erhält 0,54 kg/l·h einer 75 Gew.-% Lösung von tert-Butylperoxyisobutyrat in Isododecan (86,0 % bezogen auf Isobutyroylchlorid).

### Beispiel 8 (erfindungsgemäß)

### Herstellung von tert-Butylperoxyisobutyrat

Die Umsetzung erfolgt in einer Reaktoranordnung aus einem ersten Reaktor vom Typ E mit einem nachgeschalteten Reaktor vom Typ C. Der erste Reaktor wird vor Reaktionsbeginn mit einer Lösung von 24,4 Gew.-% tert-Butylhydroperoxid und 12,7 Gew.-% Kaliumhydroxid in Wasser gefüllt. Dann werden dem ersten Reaktor 33,6 kg/h einer Lösung von 70 Gew.-% tert-Butylhydroperoxid in Wasser, 27,2 kg/h einer Lösung von 45 Gew.-% Kaliumhydroxid in Wasser, 38,0 kg/h Wasser und 18,4 kg/h Isobutyroylchlorid zugeführt. Durch Kühlen mit Kühlwasser wird die Innentemperatur im ersten Reaktor auf 11 °C und im zweiten Reaktor auf 12 °C gehalten. Die dem zweiten Reaktor entnommene Reaktionsmischung wird mit 8,0 kg/h Isododecan gemischt und die organische Phase wird abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit einer 1 Gew.-% Lösung von Natriumhydrogencarbonat in Wasser gewaschen und danach durch Strippen in einer Füllkörperkolonne bei 20 °C und 47 mbar getrocknet. Man erhält 33,4 kg/h einer 75 Gew.-% Lösung von tert-Butylperoxyisobutyrat in Isododecan (92,8 % bezogen auf Isobutyroylchlorid). Die Raum-Zeit-Ausbeute liegt bei 2,69 kg/l·h.

### Beispiel 9 (nicht erfindungsgemäß)

### Herstellung von tert-Butylperoxybenzoat

Die Umsetzung erfolgt in einer Reaktoranordnung aus einem ersten Reaktor vom Typ D mit zwei in Reihe nachgeschalteten Reaktoren vom Typ B. Dem ersten Reaktor werden 1,72 kg/l·h einer 19,5 Gew.-% Lösung von tert-Butylhydroperoxid und 9,5 Gew.-% Natriumhydroxid in Wasser und 0,49 kg/l·h Benzoylchlorid zugeführt. Die Dosierraten beziehen sich dabei auf das Gesamtvolumen der Reaktoranordnung in Litern. Durch Kühlen mit Kühlwasser wird die Innentemperatur in allen Reaktoren auf 25 °C gehalten. Aus der dem dritten Reaktor entnommenen Reaktionsmischung wird die organische Phase abgetrennt, nacheinander mit einer 8 Gew.-% Lösung von Natriumhydroxid in Wasser, mit einer essigsauren Lösung von 10 Gew.-% Natriumsulfit in Wasser und mit entsalztem Wasser gewaschen und danach mit Calciumchlorid getrocknet. Man erhält 0,61 kg/l·h tert-Butylperoxybenzoat (89,9 % bezogen auf Benzoylchlorid).

### Beispiel 10 (erfindungsgemäß)

### Herstellung von tert-Butylperoxybenzoat

Die Umsetzung erfolgt in einer Reaktoranordnung aus einem ersten Reaktor vom Typ E mit einem nachgeschalteten Reaktor vom Typ C. Der erste Reaktor wird vor Reaktionsbeginn mit einer Lösung von 19,4 Gew.-% tert-Butylhydroperoxid und 8,9 Gew.-% Natriumhydroxid in Wasser gefüllt. Dann werden dem ersten Reaktor 18,4 kg/h einer Lösung von 70 Gew.-% tert-Butylhydroperoxid in Wasser, 11,8 kg/h einer Lösung von 50 Gew.-% Natriumhydroxid in Wasser, 35,8 kg/h Wasser und 18,0 kg/h Benzoylchlorid zugeführt. Durch Kühlen mit Kühlwasser wird die Innentemperatur im ersten Reaktor auf 12 °C und im zweiten Reaktor auf 11 °C gehalten. Aus der dem zweiten Reaktor entnommenen Reaktionsmischung wird die organische Phase abgetrennt. Die organische Phase wird nacheinander mit einer 8 Gew.-% Lösung von Natriumhydroxid in Wasser, mit einer essigsauren Lösung von 10 Gew.-% Natriumsulfit in Wasser und mit einer 1 Gew.-% Lösung von Natriumhydrogencarbonat in Wasser gewaschen und danach durch Strippen in einer Füllkörperkolonne bei 35 °C und 42 bis 44 mbar getrocknet. Man erhält 33,8 kg/h tert- tert-Butylperoxybenzoat (95,3 % bezogen auf Benzoylchlorid). Die Raum-Zeit-Ausbeute liegt bei 2,73 kg/l·h.

## Patentansprüche

1. Kontinuierliches Verfahren zur Herstellung von Acylperoxiden durch Umsetzung einer Acylverbindung aus der Reihe der Carbonsäurechloride, Carbonsäureanhydride und Chlorformiate mit einer Persauerstoffverbindung aus der Reihe der organischen Hydroperoxide und Wasserstoffperoxid, wobei die Umsetzung in mindestens zwei in Reihe geschalteten durchmischten Reaktionszonen erfolgt und der ersten Reaktionszone die Acylverbindung, die Persauerstoffverbindung und eine wässrige Lösung einer Base zugeführt werden, **dadurch gekennzeichnet, dass** die erste Reaktionszone einen Kreislauf der zweiphasigen Reaktionsmischung über einen Wärmetauscher umfasst, in dem die Reaktionsmischung gekühlt wird.

2. Verfahren nach Anspruch 1, **dadurch gekennzeichnet, dass** das Kreislauf verhältnis von umlaufender Reaktionsmischung zu der ersten Reaktionszone entnommener Rektionsmischung mindestens 2, vorzugsweise mindestens 5 beträgt.

3. Verfahren nach Anspruch 1 oder 2, **dadurch gekennzeichnet, dass** das Verhältnis zwischen der Kühlfläche des Wärmetauschers und dem Volumen der ersten Reaktionszone mindestens 1,5 m²/m³ beträgt.

4. Verfahren nach einem der vorangehenden Ansprüche, **dadurch gekennzeichnet, dass** die erste Reaktionszone ein Schlaufenreaktor ist, in dem die Reaktionsmischung mit einer Pumpe durch einen Wärmetauscher gefördert wird.

5. Verfahren nach Anspruch 4, **dadurch gekennzeichnet, dass** die Reaktionsmischung durch Kanäle oder Spalte des Wärmetauschers gefördert wird, die einen hydraulischen Durchmesser von weniger als 50 mm aufweisen.

6. Verfahren nach einem der vorangehenden Ansprüche, **dadurch gekennzeichnet, dass** der Kreislauf der ersten Reaktionszone einen Mischer umfasst und die Zugabe der Acylverbindung unmittelbar vor dem Mischer erfolgt.

7. Verfahren nach einem der vorangehenden Ansprüche, **dadurch gekennzeichnet, dass** die Zugabe der Acylverbindung in den Kreislauf der ersten Reaktionszone an einer Stelle erfolgt, an der die Strömungsgeschwindigkeit mehr als 0,5 m/s beträgt.

8. Verfahren nach einem der vorangehenden Ansprüche, **dadurch gekennzeichnet, dass** das hergestellte Acylperoxid flüssig ist und die Umsetzung ohne Zusatz eines Lösungsmittels erfolgt.

9. Verfahren nach einem der vorangehenden Ansprüche, **dadurch gekennzeichnet, dass** die Umsetzung in mehr als zwei in Reihe geschalteten durchmischten Reaktionszonen erfolgt und die zweite und die nachfolgenden Reaktionszonen die Form eines Rührzellenreaktors aufinreisen.

10. Verfahren nach einem der vorangehenden Ansprüche, **dadurch gekennzeichnet, dass** die Acylverbindung ein Carbonsäurechlorid aus der Reihe Acetylchlorid, Propionylchlorid, Butyrylchlorid, Isobutyrylchlorid, Valeroylchlorid, 2-Methylbutyrylchlorid, Pivaloylchlorid, 2-Methylpentanoylchlorid, 2-Ethylbutyrylchlorid, 2-Ethylhexanoylchlorid, Nonanoylchlorid, 2,4,4-Trimethylpentanoylchlorid, 3,5,5-Trimethylhexanoylchlorid, Decanoylchlorid, Neodecanoylchlorid, Lauroylchlorid, Benzoylchlorid, 2-Methylbenzoylchlorid, 4-Methylbenzoylchlorid, 4-Chlorbenzoylchlorid, 2,4-Dichlorbenzoylchlorid und Naphthoylchlorid ist.

11. Verfahren nach einem der vorangehenden Ansprüche, **dadurch gekennzeichnet, dass** die Acylverbindung ein Chlorformiat aus der Reihe Methylchlorformiat, Ethylchlorformiat, n-Propylchlorformiat, Isopropylchlorformiat, n-Butylchlorformiat, sec-Butylchlorformiat, 2-Ethylhexylchlorformiat, Isotridecylchlorformiat, Myristylchlorformiat, Cetylchlorformiat, Stearylchlor formiat, Cyclohexylchlorformiat, 4-tert-Butylcyclohexylchlorformiat, Benzylchlorformiat und 2-Phenoxyethylchlorformiat ist.

12. Verfahren nach einem der vorangehenden Ansprüche, **dadurch gekennzeichnet, dass** die Persauerstoffverbindung ein organisches Hydroperoxid aus der Reihe tert-Butylhydroperoxid, tert-Amylhydroperoxid, 1,1,3,3-Tetramethylbutylhydroperoxid, 2,5-Dimethylhexan-2,5-dihydroperoxid, 2,5-Dimethyl-3-hexin-2,5-Dihydroperoxid, p-Menthanhydroperoxid, Pinanhydroperoxid, Tetralinhydroperoxid, Cumolhydroperoxid, 4-tert-Butylcumolhydroperoxid, 1,3-DÜsopropylbenzoldihydroperoxid und 1,4-Diisopropylbenzoldihydroperoxid ist.

13. Reaktionssystem zur Herstellung von organischen Peroxiden, umfassend mindestens zwei in Reihe geschaltete Reaktoren, **dadurch gekennzeichnet, dass** der erste Reaktor ein Schlaufenreaktor ist, der einen in der Schlaufe angeordnetem Wärmetauscher und eine Pumpe zum Fördern der Reaktionsmischung durch den Wärmetauscher aufweist, dass das Verhältnis zwischen der Kühlfläche des Wärmetauschers und dem Volumen des Schlaufenreaktors mindestens 1,5 m²/m³ beträgt.

14. Reaktionssystem nach Anspruch 13, **dadurch gekennzeichnet, dass** der Wärmetauscher Kanäle oder Spalte zum Durchleiten der Reaktionsmischung umfasst, die einen hydraulischen Durchmesser von weniger als 50 mm aufweisen.

15. Reaktionssystem nach einem der Ansprüche 13 oder 14, **dadurch gekennzeichnet, dass** es einen nach dem Schlaufenreaktor angeordneten Rührzellenreaktor umfasst.

## Claims

1. A continuous process for preparing acyl peroxides by reacting an acyl compound from the group of carboxylic acid chlorides, carboxylic anhydrides and chloroformates with a peroxy compound from the group of organic hydroperoxides and hydrogen peroxide, the reaction proceeding in at least two mixed reaction zones connected in series and the acyl compound, the peroxy compound and an aqueous solution of a base being supplied to the first reaction zone, **characterised in that** the first reaction zone comprises circulation of the biphasic reaction mixture through a heat exchanger in which the reaction mixture is cooled.

2. A process according to claim 1, **characterised in that** the circulation ratio of circulating reaction mixture to reaction mixture withdrawn from the first reaction zone is at least 2, preferably at least 5.

3. A process according to claim 1 or 2, **characterised in that** the ratio between the cooling area of the heat exchanger and the volume of the first reaction zone is at least 1.5 m²/m³.

4. A process according to one of the preceding claims, **characterised in that** the first reaction zone is a loop reactor in which the reaction mixture is conveyed through a heat exchanger with a pump.

5. A process according to claim 4, **characterised in that** the reaction mixture is conveyed through channels or gaps of the heat exchanger which have a hydraulic diameter of less than 50 mm.

6. A process according to one of the preceding claims, **characterised in that** the circuit of the first reaction zone comprises a mixer and the acyl compound is introduced immediately upstream of the mixer.

7. A process according to one of the preceding claims, **characterised in that** the acyl compound is introduced into the circuit of the first reaction zone at a point at which the flow velocity is more than 0.5 m/s.

8. A process according to one of the preceding claims, **characterised in that** the acyl peroxide prepared is liquid and the reaction proceeds without addition of a solvent.

9. A process according to one of the preceding claims, **characterised in that** the reaction proceeds in more than two mixed reaction zones connected in series and the second and subsequent reaction zones have the form of a stirred-cell reactor.

10. A process according to one of the preceding claims, **characterised in that** the acyl compound is an acid chloride from the group of acetyl chloride, propionyl chloride, butyryl chloride, isobutyryl chloride, valeroyl chloride, 2-methylbutyryl chloride, pivaloyl chloride, 2-methylpentanoyl chloride, 2-ethylbutyryl chloride, 2-ethylhexanoyl chloride, nonanoyl chloride, 2,4,4-trimethylpentanoyl chloride, 3,5,5-trimethylhexanoyl chloride, decanoyl chloride, neodecanoyl chloride, lauroyl chloride, benzoyl chloride, 2-methylbenzoyl chloride, 4-methylbenzoyl chloride, 4-chlorobenzoyl chloride, 2,4-dichlorobenzoyl chloride and naphthoyl chloride.

11. A process according to one of the preceding claims, **characterised in that** the acyl compound is a chloroformate from the group of methyl chloroformate, ethyl chloroformate, n-propyl chloroformate, isopropyl chloroformate, n-butyl chloroformate, sec.-butyl chloroformate, 2-ethylhexyl chloroformate, isotridecyl chloroformate, myristyl chloroformate, cetyl chloroformate, stearyl chloroformate, cyclohexyl chloroformate, 4-tert.-butylcyclohexyl chloroformate, benzyl chloroformate and 2-phenoxyethyl chloroformate.

12. A process according to one of the preceding claims, **characterised in that** the peroxy compound is an organic hydroperoxide from the group of tert.-butyl hydroperoxide, tert.-amyl hydroperoxide, 1,1,3,3-tetramethylbutyl hydroperoxide, 2,5-dimethylhexane 2,5-dihydroperoxide, 2,5-dimethyl-3-hexyne 2,5-dihydroperoxide, p-menthane hydroperoxide, pinane hydroperoxide, tetralin hydroperoxide, cumene hydroperoxide, 4-tert.-butylcumene hydroperoxide, 1,3-diisopropylbenzene dihydroperoxide and 1,4-diisopropylbenzene dihydroperoxide.

13. A reaction system for preparing organic peroxides, comprising at least two reactors connected in series, **characterised in that** the first reactor is a loop reactor which has a heat exchanger arranged in the loop and a pump for conveying the reaction mixture through the heat exchanger and **in that** the ratio between the cooling area of the heat exchanger and the volume of the first reaction zone is at least 1.5 m²/m³.

14. A reaction system according to claim 13, **characterised in that** the heat exchanger comprises channels or gaps for passage of the reaction mixture which have a hydraulic diameter of less than 50 mm.

15. A reaction system according to one of claims 13 or 14, **characterised in that** it comprises a stirred-cell reactor arranged downstream of the loop reactor.

## Revendications

1. Procédé continu de production de peroxydes d'acyle par réaction d'un composé acyle de la série des chlorures d'acide carboxylique, anhydrides d'acide carboxylique et chloroformiate avec un composé peroxygéné choisi dans le groupe formé par les hydroperoxydes organiques et le peroxyde d'hydrogène, la réaction s'effectuant dans au moins deux zones de réaction agitées, montées en série, la première zone de réaction étant alimentée avec le composé acyle, le composé peroxygéné et une solution aqueuse d'une base, **caractérisé en ce que** la première zone de réaction comprend une circulation du mélange réactionnel à deux phases sur un échangeur de chaleur dans lequel le mélange réactionnel est refroidi.

2. Procédé selon la revendication 1, **caractérisé en ce que** le rapport de circulation du mélange réactionnel en circulation au mélange réactionnel retiré de la première zone de réaction est d'au moins 2, de préférence d'au moins 5.

3. Procédé selon la revendication 1 ou 2, **caractérisé en ce que** le rapport entre la zone de refroidissement de l'échangeur de chaleur et le volume de la première zone de réaction est d'au moins 1,5 m²/m³.

4. Procédé selon l'une des revendications précédentes, **caractérisé en ce que** la première zone de réaction est un réacteur à boucle dans lequel le mélange réactionnel est acheminé par une pompe en passant par un échangeur de chaleur.

5. Procédé selon la revendication 4, **caractérisé en ce que** le mélange réactionnel est acheminé par des canaux ou des fentes de l'échangeur de chaleur qui présentent un diamètre hydraulique inférieur à 50 mm.

6. Procédé selon l'une des revendications précédentes, **caractérisé en ce que** la circulation de la première zone de réaction comprend un mélangeur et l'addition du composé acyle s'effectue immédiatement avant le mélangeur.

7. Procédé selon l'une des revendications précédentes, **caractérisé en ce que** l'introduction du composé acyle dans la circulation de la première zone de réaction s'effectue à un endroit dans lequel la vitesse d'écoulement est supérieure à 0,5 m/s.

8. Procédé selon l'une des revendications précédentes, **caractérisé en ce que** le peroxyde d'acyle produit est liquide et la réaction s'effectue sans addition d'un solvant.

9. Procédé selon l'une des revendications précédentes, **caractérisé en ce que** la réaction s'effectue dans plus de deux zones de réaction agitées, montées en série, et **en ce que** la deuxième zone de réaction et les suivantes se présentent sous la forme d'un réacteur à cellules d'agitation.

10. Procédé selon l'une des revendications précédentes, **caractérisé en ce que** le composé acyle est un chlorure d'acide carboxylique de la série des chlorure d'acétyle, chlorure de propionyle, chlorure de butyryle, chlorure d'isobutyryle, chlorure de valéroyle, chlorure de 2-méthylbutyryle, chlorure de pivaloyle, chlorure de 2-méthylpentanoyle, chlorure de 2-éthylbutyryle, chlorure de 2-éthylhexanoyle, chlorure de nonanoyle, chlorure de 2,4,4-triméthylpentanoyle, chlorure de 3,5,5-triméthylhexanoyle, chlorure de décanoyle, chlorure de néodécanoyle, chlorure de lauroyle, chlorure de benzoyle, chlorure de 2-méthyl-benzoyle, chlorure de 4-méthylbenzoyle, chlorure de 4-chlorobenzoyle, chlorure de 2,4-dichlorobenzoyle et chlorure de naphtoyle.

11. Procédé selon l'une des revendications précédentes, **caractérisé en ce que** le composé acyle est un chloroformiate de la série des chloroformiate de méthyle, chloroformiate d'éthyle, chloroformiate de n-propyle, chloroformiate d'isopropyle, chloroformiate de n-butyle, chloroformiate de sec-butyle, chloroformiate de 2-éthylhexyle, chloroformiate d'isotridécyle, chloroformiate de myristyle, chloroformiate de cétyle, chloroformiate de stéaryle, chloroformiate de cyclohexyle, chloroformiate de 4-tert-butylcyclohexyle, chloroformiate de benzyle et chloroformiate de 2-phénoxyéthyle.

12. Procédé selon l'une des revendications précédentes, **caractérisé en ce que** le composé de peroxygéné est un hydroperoxyde organique de la série des hydroperoxyde de tert-butyle, hydroperoxyde de tert-amyle, hydroperoxyde de 1,1,3,3-tétraméthylbutyle, 2,5-diméthylhexane-2,5-dihydroperoxyde, 2,5-diméthyl-3-hexine-2,5-dihydroperoxyde, hydroperoxyde de p-menthane, hydroperoxyde de pinane, hydroperoxyde de tétraline, hydroperoxyde de cumène, hydroperoxyde de 4-tert-butylcumène, dihydroperoxyde de 1,3-düsopropylbenzène et dihydroperoxyde de 1,4-diisopropylebenzène.

13. Système réactionnel pour la production de peroxydes organiques, comprenant au moins deux réacteurs montés en série, **caractérisé en ce que** le premier réacteur est un réacteur à boucle qui présente un échangeur de chaleur disposé dans la boucle et une pompe pour faire passer le mélange réactionnel par l'échangeur de chaleur, **en ce que** le rapport entre la surface refroidissante de l'échangeur de chaleur et le volume du réacteur à boucle est d'au moins 1,5 m²/m³.

14. Système réactionnel selon la revendication 13, **caractérisé en ce que** l'échangeur de chaleur comprend des canaux ou des fentes pour faire passer le mélange réactionnel, qui présentent un diamètre hydraulique inférieur à 50 mm.

15. Système réactionnel selon l'une des revendications 13 ou 14, **caractérisé en ce qu'**il comprend un réacteur à cellules d'agitation disposé après le réacteur à boucle.
